# EUROPÄISCHE PATENTSCHRIFT

(11) **EP 0 886 495 B1**
(45) Veröffentlichungstag und Bekanntmachung des Hinweises auf die Patenterteilung: **05.02.2003**
(21) Anmeldenummer: 97917216.0
(22) Anmeldetag: 03.02.1997
(51) Int. Cl.: A61B 18/08, A61B 18/12

(54) **SCHNEIDVORRICHTUNG ZUR ELEKTROTOMIE**
CUTTING DEVICE FOR ELECTROTOMY
DISPOSITIF DE COUPE POUR ELECTROTOMIE

(30) Priorität: 07.02.1996 DE 19604330
(43) Veröffentlichungstag der Anmeldung: 30.12.1998
(73) Patentinhaber: Desinger, Kai, Dr., 12157 Berlin (DE)
(72) Erfinder: MÜLLER, Gerhard, D-14129 Berlin (DE); DESINGER, Kai, D-10827 Berlin (DE)
(74) Vertreter: Eisenführ, Speiser & Partner
(86) Internationale Anmeldenummer: DE9700232
(87) Internationale Veröffentlichungsnummer: WO97028751

(56) Entgegenhaltungen:
- EP-A- 0 453 071
- EP-A- 0 467 501
- EP-A- 0 646 361
- EP-A- 0 651 974
- US-A- 4 074 718

## Beschreibung

Die Erfindung betrifft eine Anordnung zur Elektrotomie gemäß dem Oberbegriff des Anspruchs 1.

Es ist seit längerem in der Chirurgie bekannt, zur Gewebekoagulation und zur Gewebetrennung hochfrequente Wechselströme im Frequenzbereich von 300 kHz bis 2 MHz zu verwenden, wodurch das behandelte Gewebe koaguliert bzw. vaporisiert wird, was als Elektrokoagulation bzw. Elektrotomie bezeichnet wird. Hierbei ist zu unterscheiden zwischen der monopolaren und der bipolaren HF-Thermotherapie.

Bei der monopolaren HF-Thermotherapie wird eine Elektrode-auch als Neutralelektrode bezeichnet - als großflächige Patientenableitung ausgelegt und in der Nähe der Eingriffsstelle am Patienten angebracht. Die eigentliche Arbeitselektrode - auch als Aktivelektrode bezeichnet - ist in ihrer Form an die jeweilige Anwendung angepaßt. So werden zur Gewebekoagulation großflächige Kugel-, Platten- oder Nadelelektroden verwendet, während bei der Gewebetrennung dünne Lanzetten- oder Schlingenelektroden eingesetzt werden.

Bei der bipolaren HF-Thermotherapie hingegen sind beide Elektroden in unmittelbarer Nähe zur Eingriffsstelle angeordnet, so daß die Wirkung des Wechselstroms auf die unmittelbare Umgebung der Eingriffsstelle begrenzt ist, wodurch ein hohes Maß an Sicherheit für Patient und Anwender gegeben ist, da Unfälle durch kapazitive Leckströme oder Verbrennung an der Neutralelektrode nicht mehr auftreten können. Ein weiterer Vorteil der bipolaren HF-Thermotherapie besteht in dem wesentlich geringeren Lastwiderstand des Gewebes zwischen den beiden Elektroden, was unter Beibehaltung des thermischen Effekts eine Verringerung der erforderlichen Generatorleistung ermöglicht.

Die HF-Thermotherapie läßt sich weiterhin einteilen nach der Lage der Elektroden in die Oberflächenkoagulation einerseits und die Tiefenkoagulation andererseits.

Bei der Oberflächenkoagulation werden in der bipolaren Technik zwei parallel angeordnete Tastelektroden verwendet, die auf die Gewebeoberfläche aufgesetzt werden, wodurch das darunterliegende Gewebe infolge des Stromflusses erhitzt und damit koaguliert wird.

Bei der Tiefenkoagulation ist es zur monopolaren Elektrotomie bekannt, Nadel-, Lanzetten- oder Schlingenelektroden zu verwenden. Hierbei müssen an der Aktivelektrode elektrische Lichtbögen generiert werden, um das vor der Aktivelektrode liegende Gewebe zu vaporisieren und damit einen Gewebsschnitt zu realisieren. Bei der monopolaren Technik ist dies verhältnismäßig einfach, da es hier nur einer bestimmten Feldstärke bedarf, um an der Aktivelektrode einen Funkenüberschlag auszulösen. Die bipolare Technik stellt dagegen größere Anforderungen an die Konzeption der Elektrodenkonfiguration, da die physikalischen Vorgänge hierbei nicht einfach zu beherrschen sind. So neigen bipolare Elektrodenanordnungen wegen der notwendigen Miniaturisierung der Neutralelektrode zu einem Umspringen der Elektrodenzuordnung in differente und indifferente Elektrode, wodurch die Funktionsfähigkeit beeinträchtigt wird. Es sind deshalb nur wenige bipolare Elektrodenanordnungen für die Tiefenkoagulation bekannt, so beispielsweise die Kugel-Nadel-Anordnung zur laparoskopischen Elektrotomie und die bipolare Nadelelektrode, die sich unter anderem zur Myomtherapie eignet.

Diese vorbekannte bipolare Elektrodenanordnung besteht aus zwei parallel angeordneten Nadelelektroden, die in das Gewebe eingestochen werden, wodurch das zwischen den Elektroden liegende Gewebe infolge des Stromflusses erhitzt und damit koaguliert wird. Diese bipolare Elektrodenanordnung ist jedoch aufgrund ihrer Anlehnung an die monopolaren Präparationshaken nicht als Schneidgerät für die HF-Chirurgie zu sehen. Nachteilig bei den bekannten bipolaren Elektrodenanordnungen ist weiterhin die relativ aufwendige Plazierung der Elektroden durch zwei Einstichstellen. Darüber hinaus läßt sich die Feldverteilung vom Anwender nur relativ ungenau vorherbestimmen, da die relative Lage der beiden Elektroden zueinander in der Regel nicht exakt vorgegeben werden kann.

Aus der EP 0 646 361 A1 ist ein elektrochirurgisches Hochfrequenz-Instrument zum Koagulieren und/ oder Schneiden von Gewebe bekannt, bei dem das distale Ende einer bipolaren Elektrodenanordnung haken- oder spatelförmig ausgebildet ist. Die Elektroden verlaufen parallel zueinander und sind mechanisch durch eine dazwischen angeordnete Isolationsschicht zu einer Baueinheit vereinigt. Nachteilig bei dieser Elektrodenanordnung ist die relativ aufwendige Plazierung der Elektroden durch zwei Einstichstellen.

Aus der US 4,074,781 ist ein elektrochirurgisches Instrument bekannt, das zwei parallele, nebeneinander liegende und nadelförmige Elektroden aufweist. Die Elektroden sind im Bereich des Schaftes der Nadeln durch einen Isolatorkörper mechanisch verbunden. Auch diese Elektrodenanordnung muss durch zwei Einstichstellen plaziert werden.

Die EP 0 453 071 A1 zeigt ein speziell für die Verwendung bei der koronaren By-Pass-Chirurgie eingesetztes Skalpell mit einer bipolaren Elektrodenanordnung. Seine Klinge umfasst auf einem keramischen Substrat ein Metallisierungsmuster mit Spalten, die ein Schneiden an der Kante des Substrates ermöglichen. Diese Anordnung hat den Nachteil, dass sie lediglich in einem speziellen Arbeitsgebiet einsetzbar ist und beispielsweise nicht für ein einfaches, linienförmiges Schneiden geeignet ist.

Die EP 0 467 501 A1 offenbart als nächstliegender Stand der Technik eine Schneidvorrichtung zur Elektrotomie mit zwei kreisbogenförmigen Elektroden, die zur Erleichterung der Handhabung an einem proximal gelegenen Teil ihrer Längserstreckung an einem Trägerelement befestigt sind. An ihren distalen Enden sind die beiden Elektroden durch ein Isolationselement mechanisch miteinander verbunden. Diese Anordnung hat den Nachteil, dass sie auf Grund der Ausbildung der Elektroden nur schwer handhabbar ist.

Aufgabe der Erfindung ist es daher, eine bipolare Schneidvorrichtung zur Elektrotomie zu schaffen, bei der die Schnittqualität und die Handhabbarkeit verbessert sind.

Die Aufgabe wird von einer Schneidvorrichtung mit den Merkmalen des Anspruchs 1 gelöst.

Das erfindungsgemäße Schneidgerät weist eine bipolare Elektrodenanordnurig auf, wobei die beiden Elektroden zur Erleichterung der Handhabbarkeit sowie zur mechanischen Fixierung mindestens an einem proximal gelegenen Teil ihrer Längserstreckung an einem Trägerelement befestigt sind. Die beiden Elektroden sind langgestreckt und werden an ihren distalen Enden durch ein Isolationselement miteinander verbunden, um die Einhaltung eines vorgegebenen Elektrodenabstandes zu erreichen.

Die beiden Elektroden verlaufen mindestens an ihren distalen Enden koaxial zueinander, wobei das Isolationselement axial zwischen den distalen Enden der beiden Elektroden angeordnet ist und einen Außenquerschnitt aufweist, der zur Erreichung einer glatten Übergangs im wesentlichen gleich dem Außenquerschnitt der beiden Elektroden an deren distalen Enden ist. Die bipolare Elektrodenanordnung weist hierbei also im Bereich des Isolationselements einen glatten und knickfreien Übergang auf, was insbesondere bei einem linienförmigen Schneiden mit einer Schnittführung parallel zur Längsachse der Elektroden vorteilhaft ist.

In einer weiterbildenden Variante der Erfindung weisen die beiden Elektroden jeweils mindestens zwei gegeneinander abgewinkelte Elektrodenschenkel auf, die in einer gemeinsamen Ebene liegen, wobei die distalen Elektrodenschenkel der beiden Elektroden zur Ermöglichung eines linienförmigen Gewebeschnittes im wesentlichen gerade und koaxial zueinander verlaufen. Die Elektrodenanordnung weist in dieser Variante also einen geraden Bereich auf, der von den distalen Elektrodenschenkeln und dem Isolationselement gebildet wird, wodurch ein linienförmiges Schneiden mit einer Schnittführung parallel zur Längsachse der Elektroden weiter vereinfacht wird, da sich ein linienförmiger Schnittkanal ausbildet, so daß feine Inzisionen möglich sind.

In der bevorzugten Ausführungsform der Erfindung bilden die beiden Elektroden zusammen mit dem Isolationselement eine bipolare Schlingenelektrode, wobei der Zwischenraum zwischen den Elektroden bzw. den Elektrodenschenkeln im wesentlichen frei bleibt, so daß neben der linearen Schnittbewegung parallel zu der von den Elektroden bzw. Elektrodenschenkeln gebildeten Ebene auch eine Schnittbewegung rechtwinklig hierzu möglich ist.

In einer anderen Variante der Erfindung sind die beiden Elektroden bzw. die Elektrodenschenkel dagegen zur Erreichung einer hohen mechanischen Belastbarkeit und damit kleinerer Elektrodenquerschnitte im wesentlichen auf ihrer gesamten Länge an dem Trägerelement befestigt. Da die mechanische Belastbarkeit in dieser Variante nahezu ausschließlich durch das Trägerelement bestimmt wird, lassen sich sehr kleine Elektrodenquerschnitte realisieren, wodurch sich die zum Schneiden erforderliche elektrische Leistung drastisch herabsetzen läßt. Darüber hinaus bieten die hierbei einsetzbaren Elektroden mit einem Durchmesser von teilweise weniger als 250 um den Vorteil einer verbesserten Schnittführung, die mit der einer monopolaren Spatelektrode vergleichbar ist. Weiterhin ermöglichen kleine Elektrodendurchmesser und kleine Schenkellängen vorteilhaft kleinere Schnittradien, was bei der chirurgischen Präparation wichtig ist.

In der bevorzugten Ausführungsform dieser Variante ist das Trägerelement spatelförmig ausgebildet, wobei die proximalen Elektrodenschenkel entlang den Seitenkanten des Trägerelements angeordnet und an diesen befestigt sind, während die distalen Elektrodenschenkel entlang der Schnittkante des spatelförmigen Trägerelements angeordnet und an dieser angebracht sind. Die Isolation der beiden Elektroden gegeneinander kann zwischen den distalen Enden der beiden distalen Elektrodenschenkel wahlweise - wie bei den vorstehend beschriebenen Varianten der Erfindung - durch ein separates Isolationselement oder durch das Trägerelement selbst erfolgen. Hierbei ist es jedoch nicht erforderlich, daß das Isolationselement bzw. das Trägerelement den Raum zwischen den distalen Enden der Elektroden vollständig ausfüllt. Entscheidend ist vielmehr, daß die beiden distalen Enden der Elektroden durch das Isolationselement bzw. das Trägerelement mechanisch zueinander fixiert werden, was beispielsweise auch dadurch erfolgen kann, daß die beiden Elektroden auf ihrer gesamten Länge mit dem Trägerelement verbunden sind, wobei zwischen den distalen Enden der beiden Elektroden ein definierter Luftspalt verbleibt. Diese Variante der Erfindung eignet sich aufgrund der erhöhten Stabilität und der möglichen Miniaturisierung mit kleinen Elektrodendurchmessern besonders vorteilhaft für den Einsatz in der Laparoskopie oder flexiblen Endoskopie.

Bei den vorstehend beschriebenen Varianten der Erfindung ist stets eine der beiden Elektroden die aktive Elektrode-auch als differente Elektrode bezeichnet - und die andere Elektrode die Neutralelektrode - auch als indifferente Elektrode bezeichnet. Die aktive Elektrode zeichnet sich durch die Ausbildung von Funkenüberschlägen aus und wirkt somit gewebetrennend, während die Neutralelektrode lediglich als Rückleiter fungiert.

In der bevorzugten Ausführungsform der Erfindung ist die Zuordnung von Aktivelektrode und Neutralelektrode vom Benutzer bestimmbar, indem die Schneidvorrichtung zu Beginn des Schneidvorgangs asymmetrisch so aufgesetzt wird, daß eine der beiden Elektroden das Gewebe zuerst berührt. Durch ein derartiges, bewußt asymmetrisches Aufsetzen der Schneidvorrichtung wird die noch nicht aufgesetzte Elektrode zur Aktivelektrode, da hier durch die noch bestehende Luftstrecke eine wesentlich größere Feldstärke herrscht. Diese Luftstrecke wird dann infolge der großen Feldstärke von einem Lichtbogen durchschlagen, so daß ein Dampfpolster entsteht, welches das umliegende Gewebe von der Aktivelektrode abhebt, wobei Funkenüberschläge laufend den Zwischenraum zwischen dem Gewebe und der Aktivelektrode überbrücken und die gesamte Gewebefront abtasten, so daß das Gewebe quasi berührungsfrei von der Aktivelektrode getrennt wird. Wichtig ist hierbei, daß die geometrischen Verhältnisse der beiden Elektrode - also beispielsweise ihre effektive Länge - nicht zu unterschiedlich sind, damit die Elektrodenzuordnung nicht bereits durch die Elektrodenanordnung vorgegeben ist. Vorzugsweise weisen die beiden Elektroden in dieser Variante der Erfindung deshalb im Bereich des Gewebekontaktes dieselbe Länge auf, damit der Benutzer die Elektrodenzuordnung durch entsprechend asymmetrisches Aufsetzen frei vorgeben kann.

In einer anderen Variante der Erfindung ist dagegen vorgesehen, daß sich unabhängig von der Führung der Schneidvorrichtung durch den Benutzer stets eine vorgegebene Elektrodenzuordnung einstellt. Hierzu weisen die beiden Elektroden im Bereich des Gewebekontaktes stark unterschiedliche Geometrien, insbesondere unterschiedliche Längen, auf.

Andere vorteilhafte Weiterbildungen der Erfindung sind in den Unterahsprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel der Erfindung ein Schneidgerät zur Elektroto-mie mit einer bipolaren schlingenförmigen Elektrodenanordnung in perspektivischer Darstellung,
- Figur 2: das Schneidgerät aus Figur 1 in Seitenan-sicht zur Erläuterung der biophysikalischen Vorgänge an den beiden Elektroden,
- Figur 3a, 3b: ein weiteres erfindungsgemäßes Schneidgerät in Seitenansicht bzw. ein Detail des Schneidgerätes sowie,
- Figur 4a bis 4d: ein Schneidgerät zur Elektrotomie mit einer auswechselbaren Schneidspitze in perspektivischer Darstellung

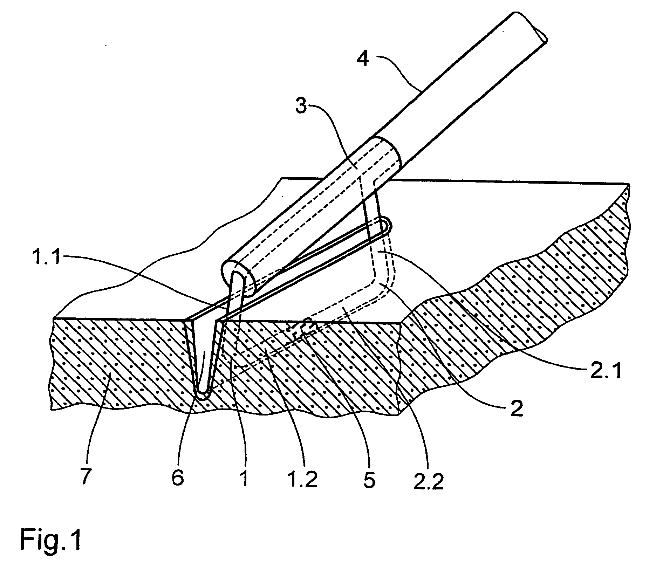

Figur 1 zeigt ein erfindungsgemäßes Schneidgerät für die Elektrotomie mit einer bipolaren Elektrodenanordnung, die im wesentlichen aus einer ersten Elektroden 1 und einer zweiten Elektrode 2 aus Edelstahldraht besteht, die zur mechanischen Fixierung an ihren proximalen Enden durch einen Hochtemperaturkleber an einem zylindrischen Trägerelement 3 befestigt sind, das an seinem den Elektroden 1, 2 abgewandten Ende ein angeformtes Handstück 4 zur manuellen Führung durch den Operateur aufweist. Die beiden Elektroden 1, 2 sind jeweils in einen proximalen Elektrodenschenkel 1.1, 2.1 und einen gegen den proximalen Elektrodenschenkel 1.1, 2.1 abgewinkelten distalen Elektrodenschenkel 1.2, 2.2 unterteilt, wobei die einzelnen Elektrodenschenkel 1.1, 1.2, 2.1, 2.2 der beiden Elektroden 1, 2 in einer gemeinsamen Ebene liegen und jeweils gerade, ohne wesentliche Krümmungen entlang ihrer Längserstreckung ausgeführt sind. An ihren distalen Enden sind die distalen Elektrodenschenkel 1.2, 2.2 der beiden Elektroden 1, 2 durch ein zylindrisches Isolationselement 5 mechanisch miteinander verbunden, so daß die beiden Elektroden 1, 2 zusammen mit dem Isolationselement 5 eine bipolare Schlingenanordnung bilden, die eine Schnittbewegung sowohl parallel als auch rechtwinklig zur Elektrodenebene ermöglicht. Besonders vorteilhaft läßt sich mit diesem Schneidgerät jedoch eine lineare Schnittbewegung ausführen, da sich auf diese Weise ein linearer, furchenförmiger Schnittkanal 6 im Gewebe 7 ausbildet, durch den das Schneidgerät mit den beiden distalen Elektrodenschenkeln 1.2, 2.2 hindurchgezogen werden kann.

Ein weiterer Vorteil der Schlingenanordnung ist darin zu sehen, daß für den Operateur die freie Sicht auf die unmittelbare Eingriffsstelle erhalten bleibt. Darüber hinaus wird durch das Isolationselement 5 auch die Einhaltung eines vorgegebenen Elektrodenabstandes sichergestellt.

Zur Erleichterung einer linearen Schnittbewegung parallel zur Elektrodenebene sind die distalen Elektrodenschenkel 1.2, 2.2 der beiden Elektroden 1, 2 zusammen mit dem zylindrischen Isolationselement 5 koaxial angeordnet, wobei das Isolationselement 5 denselben Außenquerschnitt aufweist wie die distalen Elektrodenschenkel 1.2, 2.2, um einen glatten und absatzlosen Übergang zwischen den distalen Elektrodenschenkeln 1.2, 2.2 und dem Isolationselement 5 zu erreichen.

Die elektrische Ansteuerung der beiden Elektroden 1, 2 erfolgt durch einen separaten Hochfrequenzgenerator, der über jeweils eine, durch einen Hohlkanal in dem Trägerelement 3 hindurchgeführte Zuleitung mit den beiden Elektroden 1, 2 verbunden ist.

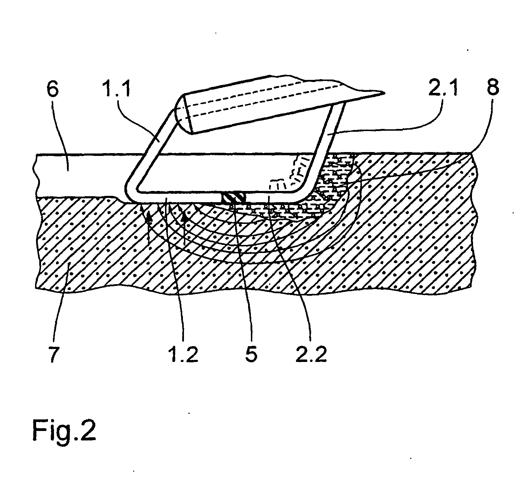

Die biophysikalischen Vorgänge während der Schnittbewegung werden durch die in Figur 2 dargestellte Seitenansicht des Schneidgeräts verdeutlicht und nachfolgend detailliert beschrieben.

Zu Beginn des Schneidvorgangs wird der Schnitt initialisiert, wobei die Zuordnung der beiden Elektroden in Aktivelektrode (differente Elektrode) und Neutralelektrode (indifferente Elektrode) vom Benutzer gewählt wird. Hierzu setzt der Operateur das Schneidgerät derart asymmetrisch auf das Gewebe 7 auf, daß die entgegen der Schnittrichtung gelegene Elektrode 2 das Gewebe 7 zuerst berührt. Durch ein derartiges, bewußt asymmetrisches Aufsetzen des Schneidgeräts wird sich der noch nicht aufgesetzte Elektrodenschenkel 2.2 zur Aktivelektrode ausbilden, da hier durch die noch bestehende Luftstrecke eine wesentlich größere Feldstärke herrscht. Diese Luftstrecke wird dann infolge der großen Feldstärke von einem Lichtbogen durchschlagen, so daß ein Dampfpolster 8 entsteht, welches das umliegende Gewebe 7 von der Elektrode 2 abhebt, wobei Funkenüberschläge laufend den Zwischenraum zwischen dem Gewebe 7 und der aktiven Elektrode 2 überbrücken und die gesamte Gewebefront abtasten, so daß das Gewebe 7 quasi berührungsfrei von der aktiven Elektrode 2 getrennt wird.

Zum distalen Ende des aktiven Elektrodenschenkels 2.2 hin verkleinert sich das Dampfpolster 8 aufgrund abnehmender Funkenüberschläge in diesem Bereich. Im Bereich des neutralen Elektrodenschenkels 1.2 wird das Gewebe 7 dann durch die Anpreßkraft des Schneidgeräts bzw. durch die Reaktionskraft des Gewebes 7 an die Neutralelektrode 1 angedrückt, was einen guten elektrischen Kontakt zwischen dem Gewebe 7 und der Neutralelektrode 1 und damit eine eindeutige Zuordnung der Stromdichteverteilung bewirkt. Selbst bei einer sich stark verkleinernden effektiven Kontaktfläche der Neutralelektrode 1 bleibt die Zuordnung von aktiver und neutraler Elektrode zunächst bestehen. Erst wenn die Kontaktfläche der Neutralelektrode 1 einen bestimmten Minimalwert unterschreitet kann es zum Umspringen des Lichtbogens und damit zu einem Wechsel der Elektrodenzuordnung kommen. Dieser Fall kann beispielsweise eintreten, wenn der indifferente Elektrodenschenkel 1.2 fast ganz aus dem Schnittkanal herausgezogen wird oder wenn die Durchzugsgeschwindigkeit des Schneidgeräts gleich Null ist. Bei einer normalen Handhabung des dargestellten Schneidgeräts konnte ein Umspringen des Lichtbogens jedoch nicht beobachtet werden.

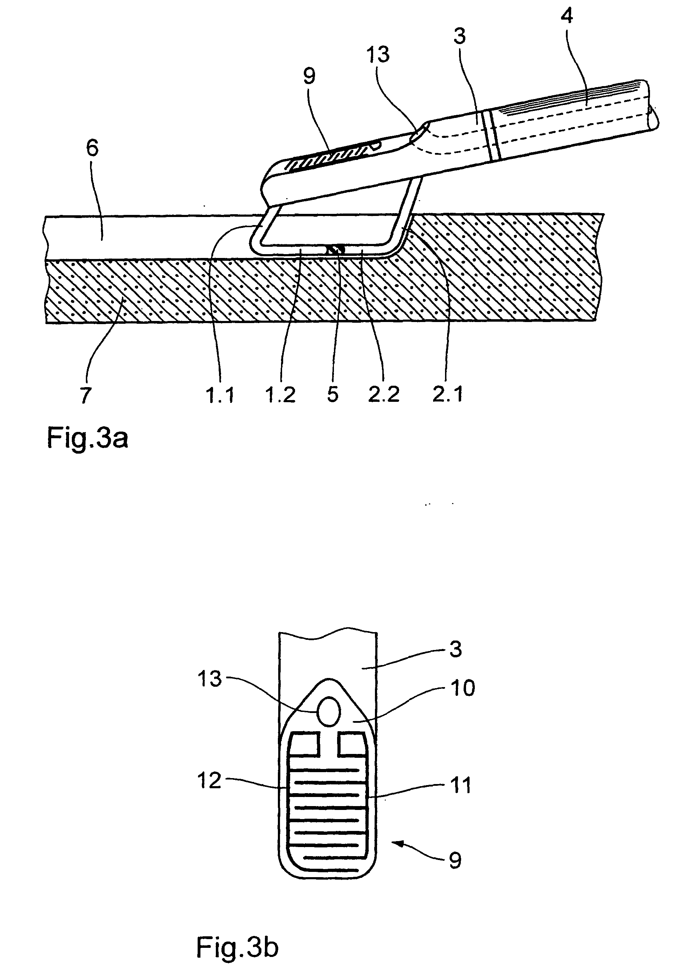

Figur 3a zeigt ebenfalls ein Schneidgerät zur Gewebetrennung, das mit dem vorstehend beschriebenen und in den Figuren 1 und 2 dargestellten Schneidgerät weitgehend übereinstimmt, jedoch zusätzlich eine Koagulationseinrichtung 9 zur Blutungsstillung aufweist, die detailliert in Figur 3b in einer Aufsicht dargestellt ist. Wegen der weitgehenden baulichen Übereinstimmung mit dem bereits vorstehend beschriebenen Schneidgerät sind funktionsgleiche Bauelemente in den Figuren 1 und 2 sowie in Figur 3a und 3b mit denselben Bezugszeichen versehen, so daß diesbezüglich zur Erläuterung auf die vorstehenden Ausführungen verwiesen wird.

Die Koagulationseinrichtung 9 ist an der Oberseite des Trägerelements 3 in einer Vertiefung angebracht, so daß der Operateur zur Elektrokoagulation lediglich das Schneidgerät um seine Längsachse drehen und die Koagulationseinrichtung 9 auf das Gewebe auflegen muß, um eine beim Gewebeschneiden auftretende Blutung zu stoppen. Auf diese Weise ist es möglich, im schnellen Wechsel Gewebe 7 zu trennen und die dabei auftretenden Blutungen durch Elektrokoagulation zu stillen. Die Koagulationseinrichtung 9 besteht im wesentlichen aus einem flachen Elektrodenträger 10 aus elektrisch isolierendem Material sowie einer ersten Elektrode 11 und einer zweiten Elektrode 12, die aus einer Silber-Panadium-Legierung bestehen und während der Herstellung mit einer feinen Düse im flüssigen Zustand auf den Elektrodenträger 10 aufgebracht und anschließend eingebrannt werden. Durch ein anschließendes Vernickeln der Oberfläche wird das Elektrodenmaterial an der Oberfläche gehärtet, um eine gute Standfestigkeit zu erreichen.

Die beiden Elektroden 11, 12 weisen mehrere parallel verlaufenden linienförmige Elektrodenbahnen auf, die mäanderförmig ineinander greifen, um die wirksame Elektrodenoberfläche zu vergrößern und eine möglichst effektive Elektrokoagulation zu erreichen.

Weiterhin weist das Trägerelement 3 zur Zuführung einer Spülflüssigkeit einen durchgehenden Hohlkanal auf, der in einer Öffnung 13 in der Koagulationseinrichtung 9 mündet, durch welche die Spülflüssigkeit in das zu koagulierenden Gewebe 7 abgegeben werden kann. Hierdurch ist es möglich, eine Austrocknung des Gewebes 7 zu verhindern, was zu einer Verbesserung der elektrischen Ankopplung der Koagulationseinrichtung 9 an das Gewebe 7 beiträgt.

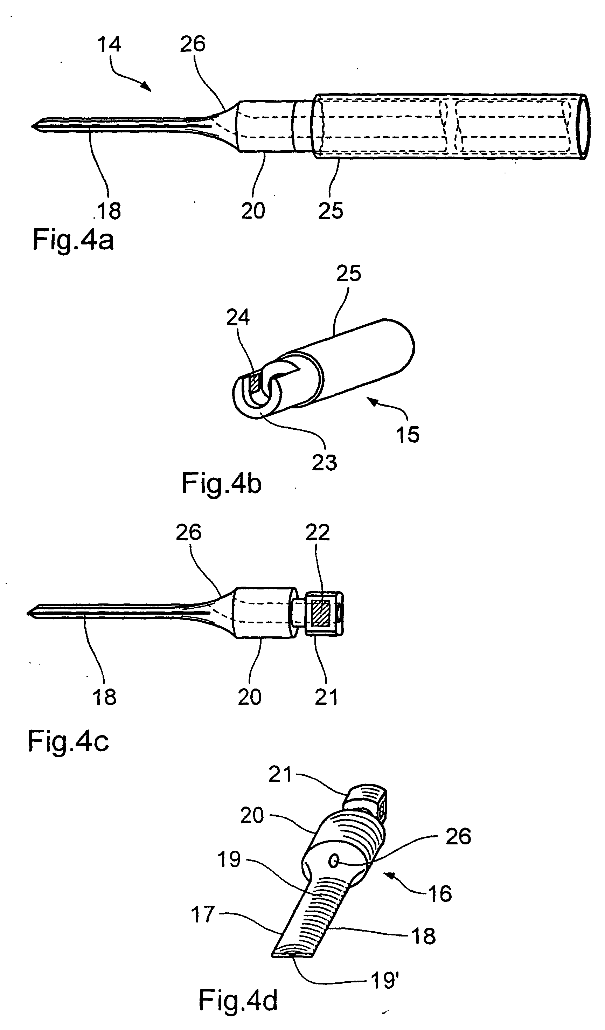

Figur 4a zeigt ein weiteres erfindungsgemäßes Schneidgerät 14 zur Elektrotomie, das im wesentlichen aus einem in Figur 4b dargestellten Handhabungsteil 15 und einer in Figur 4c und 4d gezeigten auswechselbaren Schneidspitze 16 besteht.

Zur elektro-thermischen Gewebetrennung weist die Schneidspitze 16 eine erste Elektrode 17 und eine zweite Elektrode 18 auf, die aus Edelstahldraht mit einem Durchmesser von 200 µm bestehen und jeweils in einen proximalen Elektrodenschenkel mit einer Länge von 2,5 mm und einen rechtwinklig abgewinkelten distalen Elektrodenschenkel unterteilt sind, wobei die proximalen Elektrodenschenkel entlang den Seitenkanten eines spatelförmigen Trägerelements 19 angeordnet und an diesem befestigt sind, wohingegen die distalen Elektrodenschenkel entlang der Schnittkante des spatelförmigen Trägerelements 19 angeordnet und an dieser befestigt sind. Die Elektroden 17, 18 sind hierzu seitlich in entsprechende Vertiefungen in dem elektrisch isolierenden Trägerelement 19 eingelassen, wobei das Trägerelement 19 den Raum zwischen den distalen Enden der beiden distalen Elektrodenschenkel 1.2, 2.2 ausfüllt und ein Isolationselement 19' mit einem vorgegebenen Elektrodenabstand bildet. Das aus Keramik bestehende Trägerelement 19 dient hierbei zum einen zur mechanischen Fixierung und Führung der Elektroden 17, 18 und zum anderen zur elektrischen Isolation der Elektroden 17, 18 gegeneinander.

Weiterhin weist das Trägerelement 19 auf der den Elektroden 17, 18 abgewandten Seite einen zylindrischen Schaft auf, an den ein Sattelstück 21 angeformt ist, welches eine mechanische und elektrische Verbindung mit dem Handhabungsteil 15 ermöglicht, wobei die Verbindung zwischen der Schneidspitze 16 und dem Handhabungsteil 15 lösbar ist, um einen Wechsel der Schneidspitze 16 zu ermöglichen. Die elektrische Verbindung der Schneidspitze 16 mit dem Handhabungsteil 15 erfolgt durch zwei Kontaktfahnen 22, die mit den beiden Elektroden 17, 18 der Schneidspitze 16 verbunden sind.

Zur Aufnahme der Schneidspitze 16 weist das Handhabungsteil 15 ein entsprechend formangepaßtes Aufnahmeelement 23 auf, in dem ebenfalls zwei Kontaktfahnen 24 angeordnet sind, die über Zuleitungen eine Verbindung mit einem separaten Hochfrequenzgenerator ermöglichen, wobei die Zuleitungen durch einen Hohlkanal in dem Handhabungsteil 15 hindurchgeführt sind. Weiterhin weist das Handhabungsteil 15 eine zylindrische Hülse 25 auf, die in axialer Richtung verschoben werden kann. Zur Montage oder zum Auswechseln der Schneidspitze 16 wird die Hülse 25 nach hinten geschoben, um das Aufnahmeelement des Handhabungsteils 15 freizugeben. Anschließend wird das Sattelstück 21 der gewünschten Schneidspitze 16 in das Aufnahmeelement 23 eingelegt und die Hülse 25 wieder in ihre Ausgangslage geschoben, um die Verbindung zwischen der Schneidspitze 16 und dem Handhabungsteil 15 zu verriegeln. Darüber hinaus kann die Hülse 25 auch über die Schneidspitze 16 geschoben werden, um diese beim Einführen über einen Trockar vor mechanischen Beschädigungen zu schützen.

Darüber hinaus weist das Handhabungsteil 15 einen zentral angeordneten, durchgehenden Hohlkanal auf, der in der Schneidspitze 16 fortgesetzt ist und in einer Öffnung 26 an der Oberseite der Schneidspitze 16 mündet. Hierdurch ist es zum einen möglich, eine Spülflüssigkeit in das Gewebe zu leiten, um eine elektrische Austrocknung des Gewebes zu verhindern. Zum anderen kann so Gewebesubstanz oder Flüssigkeit aus dem Gewebe abgesaugt werden. Die axial verschiebbare Hülse 25 ermöglicht hierbei vorteilhaft eine Fokussierung des Spülmittel- bzw. Saugstroms, indem die Hülse über die Öffnung 26 geschoben wird.

## Patentansprüche

1. Schneidvorrichtung (1 bis 7, 14) zur Elektrotomie mit zwei zueinander beabstandet angeordneten und elektrisch gegeneinander isolierten langgestreckten Elektroden (1, 2, 17, 18) zum Anschluß an einen Hochfrequenzgenerator,
die zur Erleichterung der Handhabung mindestens an einem proximal gelegenen Teil ihrer Längserstreckung an einem Trägerelement (3) befestigt und an ihren distalen Enden zur Erreichung eines vorgegebenen Elektrodenabstands durch ein Isolationselement (5, 19') mechanisch miteinander verbunden sind,
**dadurch gekennzeichnet, daß** die beiden Elektroden (1, 2, 17, 18) mindestens an ihren distalen Enden im wesentlichen koaxial zueinander verlaufen, wobei das Isolationselement (5, 19') axial zwischen den distalen Enden der beiden Elektroden (1, 2, 17, 18) angeordnet ist und einen Außenquerschnitt aufweist, der zur Erreichung eines glatten Übergangs im wesentlichen gleich dem Außenquerschnitt der beiden Elektroden (1, 2, 17, 18) an deren distalen Enden ist.

2. Schneidvorrichtung (1 bis 7, 14) nach Anspruch 1, **dadurch gekennzeichnet, daß** die beiden Elektroden (1, 2, 17, 18) jeweils mindestens zwei gegeneinander abgewinkelte Elektrodenschenkel (1.1, 1.2, 2.1, 2.2) aufweisen, die in einer gemeinsamen Ebene liegen, wobei die distalen Elektrodenschenkel (1.2, 2.2) der beiden Elektroden (1, 2, 17, 18) zur Ermöglichung eines linienförmigen Gewebeschnittes im wesentlichen geradlinig und koaxial zueinander verlaufen.

3. Schneidvorrichtung (1 bis 7) nach Anspruch 2, **dadurch gekennzeichnet,** die distalen Elektrodenschenkel (1.2, 2.2) der beiden Elektroden (1, 2) zur eindeutigen Festlegung von aktiver und neutraler Elektrode unterschiedlich lang sind und/oder unterschiedliche Außenquerschnitte aufweisen.

4. Schneidvorrichtung (1 bis 7) nach einem der vorhergehenden Ansprüche, **dadurch gekennzeichnet, daß** die beiden Elektroden (1, 2) zusammen mit dem Isolationselement (5) eine Schlinge bilden.

5. Schneidvorrichtung (14) nach einem der Ansprüche 1 bis 4, **dadurch gekennzeichnet, daß** die beiden Elektroden (17, 18) zur Erreichung einer großen mechanischen Belastbarkeit und damit kleiner Elektrodenquerschnitte im wesentlichen auf ihrer gesamten Länge an dem Trägerelement (19) befestigt sind.

6. Schneidvorrichtung (14) nach Anspruch 2 und Anspruch 5, **dadurch gekennzeichnet, daß** das Trägerelement (19) spatelförmig mit zwei Seitenkanten und einer Schnittkante ausgebildet ist, wobei die proximalen Elektrodenschenkel (1.1, 2.1) entlang den Seitenkanten des Trägerelements (19) angeordnet und an diesen befestigt und die distalen Elektrodenschenkel (1.2, 2.2) entlang der Schnittkante des spatelförmigen Trägerelements (19) angeordnet und an dieser befestigt sind.

7. Schneidvorrichtung (14) nach Anspruch 5 oder 6, **dadurch gekennzeichnet, daß** das Trägerelement (19) zur Zuführung eines Spülmittels und/oder zur Absaugung von Körpersubstanz einen durchgehenden Hohlkanal aufweist, der zur Abgabe der Spülmittel und/oder zur Absaugung der Körpersubstanz in einer Öffnung (26) mündet.

8. Schneidvorrichtung (14) nach einem der Ansprüche 5 bis 7, **dadurch gekennzeichnet, daß** das Trägerelement (19) einen Schaft (20) aufweist, der von einer Hülse (25) umgeben ist, wobei der Innenquerschnitt der Hülse (25) derart an den Außenquerschnitt des Schaftes (20) angepaßt ist, daß die Hülse (25) zur Fokussierung der Spülmittelströmung und/oder des Saugstroms axial verschiebbar ist.

9. Schneidvorrichtung (14) nach einem der Ansprüche 5 bis 8, **dadurch gekennzeichnet, daß** zur handgeführten Bedienung ein Handhabungsteil (15) vorgesehen ist, das mit dem Trägerelement (19) durch eine lösbare Steckverbindung (21, 23) mechanisch und elektrisch verbunden ist.

10. Schneidvorrichtung (14) nach Anspruch 9, **dadurch gekennzeichnet, daß** die Steckverbindung ein an das Trägerelement (19) angeformtes oder an diesem befestigtes Sattelstück (21) und ein an dem Handhabungsteil (15) angeformtes oder an diesem befestigtes Aufnahmeelement (23) aufweist, das in seiner Form an das Sattelstück (21) angepaßt ist, wobei zur Fixierung der Steckverbindung eine Hülse (25) vorgesehen ist, die das Handhabungsteil (15) umgibt und in axialer Richtung über das Sattelstück (21) und das Aufnahmeelement (23) verschiebbar ist.

11. Schneidvorrichtung (1 bis 7, 14) nach einem der vorhergehenden Ansprüche, **dadurch gekennzeichnet, daß** das Isolationselement (5) aus Keramik besteht.

12. Schneidvorrichtung (1 bis 7, 14) nach einem der vorhergehenden Ansprüche, **dadurch gekennzeichnet, daß** zur Gewebekoagulation bei Blutungen eine dritte Elektrode (11) und eine vierte Elektrode (12) jeweils zum Anschluß an einen Hochfrequenzgenerator vorgesehen sind, die auf der Oberfläche des Trägerelements (3) oder eines flächigen Isolationskörpers (10) zueinander beabstandet und elektrisch gegeneinander isoliert angebracht sind, wobei die dritte Elektrode (11) und die vierte Elektrode (12) mindestens abschnittsweise langgestreckt und zur Vergrößerung der effektiven Oberfläche entlang ihrer Längserstreckung nebeneinander angeordnet sind.

13. Schneidvorrichtung (1 bis 7, 14) nach Anspruch 12, **dadurch gekennzeichnet, daß** die dritte Elektrode (11) und die vierte Elektrode (12) jeweils spiralförmig geformt und ineinander eingreifend angeordnet sind.

14. Schneidvorrichtung (1 bis 7, 14) nach Anspruch 13, **dadurch gekennzeichnet, daß** die dritte Elektrode (11) und die vierte Elektrode (12) mäanderförmig angeordnet sind.

## Claims

1. A cutting device (1 to 7, 14) for electrotomy with two mutually spaced, elongate electrodes (1, 2, 17, 18), that are electrically insulated from one another, for connection to a high-frequency generator, which to facilitate operation are fixed at least at one proximally located part of their longitudinal extension to a support member (3) and to achieve a predetermined electrode gap are mechanically connected to one another by an insulating member (5, 19') at their distal ends,
**characterised in that** the two electrodes (1, 2, 17, 18) run substantially coaxially to one another at least at their distal ends, with the insulating member (5, 19') being disposed axially between the distal ends of the two electrodes (1, 2, 17, 18) and having an external cross section which, to achieve a smooth transition, is substantially equal to the external cross section of the two electrodes (1, 2, 17, 18) at their distal ends.

2. A cutting device (1 to 7, 14) according to Claim 1,
**characterised in that** the two electrodes (1, 2, 17, 18) in each case comprise at least two electrode limbs (1.1, 1.2, 2.1, 2.2) bent towards one another, which lie in a common plane, with the distal electrode limbs (1.2, 2.2) of the two electrodes (1, 2, 17, 18) running substantially rectilinearly and coaxially to one another to enable a linear tissue severance.

3. A cutting device (1 to 7) according to to Claim 2,
**characterised in that** for the clear determination of the active and neutral electrode, the distal electrode limbs (1.2, 2.2) of the two electrodes (1, 2) are of different lengths and/or have different external cross sections.

4. A cutting device (1 to 7) according to one of the preceding Claims,
**characterised in that** the two electrodes (1,2) together with the insulating member (5) form a loop.

5. A cutting device (14) according to one of Claims 1 to 4,
**characterised in that** to achieve a large mechanical stability under load and thus small electrode cross sections, the two electrodes (17, 18) are fixed substantially over their entire length to the support member (19).

6. A cutting device (14) according to Claim 2 and Claim 5,
**characterised in that** the support member (19) has a spatula-shaped construction with two side edges and a cutting edge, with the proximal electrode limbs (1.1, 2.1) being disposed along the side edges of the support member (19) and being fixed thereto and the distal electrode limbs (1.2, 2.2) being disposed along the cutting edge of the spatula-shaped support member (19) and being fixed thereto.

7. A cutting device (14) according to Claim 5 or 6,
**characterised in that** for supplying a rinsing agent and/or for aspirating body material, the support member (19) comprises a through hollow duct, which for the discharge of the rinsing agent and/or for aspirating the body material opens in an opening (26).

8. A cutting device (14) according to one of Claims 5 to 7,
**characterised in that** the support element (19) comprises a shaft (20), which is surrounded by a sleeve (25), wherein the internal cross section of the sleeve (25) is adapted to the outer cross section of the shaft (20) in such a manner that the sleeve (25) is axially displaceable to focus the flow of rinsing agent and/or the suction stream.

9. A cutting device (14) according to one of Claims 5 to 8,
**characterised in that** an operating part (15), which is mechanically and electrically connected to the support member (19) by a detachable plug-in connection (21, 23), is provided for hand-controlled operation.

10. A cutting device (14) according to Claim 9,
**characterised in that** the plug-in connection comprises a saddle piece (21) moulded on the support member (19) or fixed thereto and a housing member (23) moulded on the operating part (15) or fixed thereto, whose shape is adapted to the saddle piece (21), with a sleeve (25), which surrounds the operating part (15) and is displaceable in the axial direction over the saddle piece (21) and the housing member (23), being provided to fasten the plug-in connection.

11. A cutting device (1 to 7, 14) according to one of the preceding Claims,
**characterised in that** the insulating member (5) is made from ceramics.

12. A cutting device (1 to 7, 14) according to one of the preceding Claims,
**characterised in that** for the coagulation of tissue in the event of bleeding, a third electrode (11) and a fourth electrode (12) are in each case provided for connection to a high-frequency generator, which are mounted spaced from one another on the surface of the support member (3) or a flat insulating member (10) and electrically insulated with respect to one another, with the third electrode (11) and the fourth electrode (12) being elongated at least in some sections and being disposed next to one another along their longitudinal extension to increase the effective surface.

13. A cutting device (1 to 7, 14) according to Claim 12,
**characterised in that** the third electrode (11) and the fourth electrode (12) are helicoidal in each case and are arranged so as to mutually engage.

14. A cutting device (1 to 7, 14) according to Claim 13,
**characterised in that** the third electrode (11) and the fourth electrode (12) have a meandering arrangement.

## Revendications

1. Dispositif de coupe (1 à 7, 14) pour l'électrotomie, comportant deux électrodes allongées (1, 2, 17, 18) agencées à distance et électriquement isolées l'une de l'autre destinées au branchement à un générateur de haute fréquence, qui, afin de faciliter la manipulation, sont fixées au moins sur une partie proximale de leur extension longitudinale sur un élément de support (3) et qui sont reliées mécaniquement l'une à l'autre à leurs extrémités distales par un élément d'isolation (5, 19') pour obtenir une distance déterminée entre les électrodes, **caractérisé en ce que** les deux électrodes (1, 2, 17, 18) s'étendent sensiblement coaxialement l'une à l'autre au moins à leurs extrémités distales, l'élément d'isolation (5, 19') étant agencé axialement entre les extrémités distales des deux électrodes (1, 2, 17, 18) et présentant une section extérieure qui est sensiblement égale à la section extérieure des deux électrodes (1, 2, 17, 18) à leurs extrémités distales pour obtenir une transition lisse.

2. Dispositif de coupe (1 à 7, 14) selon la revendication 1, **caractérisé en ce que** les deux électrodes (1, 2, 17, 18) comprennent chacune au moins deux bras d'électrode (1.1, 1.2, 2.1, 2.2) coudés l'un par rapport à l'autre qui se trouvent dans un plan commun, les bras d'électrodes distaux (1.2, 2.2) des deux électrodes (1, 2, 17, 18) s'étendant sensiblement en ligne droite et coaxialement l'un par rapport à l'autre pour permettre une coupe linéaire du tissu.

3. Dispositif de coupe (1 à 7) selon la revendication 2, **caractérisé en ce que** les bras distaux (1.2, 2.2) des deux électrodes (1, 2) présentent des longueurs différentes et/ou des sections extérieures différentes pour définir clairement l'électrode active et l'électrode neutre.

4. Dispositif de coupe (1 à 7) selon l'une des revendications précédentes, **caractérisé en ce que** les deux électrodes (1, 2) forment une boucle conjointement avec l'élément d'isolation (5).

5. Dispositif de coupe (14) selon l'une des revendications 1 à 4, **caractérisé en ce que** les deux électrodes (17, 18) sont fixées sensiblement sur toute leur longueur sur l'élément de support (19) pour obtenir une grande capacité de charge mécanique et ainsi des petites sections d'électrode.

6. Dispositif de coupe (14) selon la revendication 2 et la revendication 5, **caractérisé en ce que** l'élément de support (19) est réalisé en forme de spatule comportant deux arêtes latérales et une arête coupante, les bras d'électrode proximaux (1.1, 2.1) étant agencés le long des arêtes latérales de l'élément de support (19) et fixés sur celles-ci, et les bras d'électrode distaux (1.2, 2.2) étant agencés le long de l'arête coupante de l'élément de support (19) en forme de spatule et fixés sur celle-ci.

7. Dispositif de coupe (14) selon l'une ou l'autre des revendications 5 et 6, **caractérisé en ce que** l'élément de support (19) présente un canal creux continu pour amener un produit de rinçage et/ou pour aspirer une substance corporelle, qui débouche dans une ouverture (26) pour distribuer le produit de rinçage et/ou pour aspirer la substance corporelle.

8. Dispositif de coupe (14) selon l'une des revendications 5 à 7, **caractérisé en ce que** l'élément de support (19) comprend une tige (20) qui est entourée par une douille (25), la section intérieure de la douille (25) étant adaptée à la section extérieure de la tige (20) de telle sorte que la douille (25) est mobile axialement pour focaliser l'écoulement du produit de rinçage et/ou le courant d'aspiration.

9. Dispositif de coupe (14) selon l'une des revendications 5 à 8, **caractérisé en ce qu'**il est prévu une partie de manipulation (15) pour la manipulation à la main, qui est reliée mécaniquement et électriquement à l'élément de support (19) par une liaison à enfichage détachable (21, 23).

10. Dispositif de coupe (14) selon la revendication 9, **caractérisé en ce que** la liaison à enfichage comprend une pièce d'accouplement (21) conformée ou fixée sur l'élément de support (19) et un élément de réception (23) conformé ou fixé sur la partie de manipulation (15) dont la forme est adaptée à la forme de la pièce d'accouplement (21), une douille (25) étant prévue pour solidariser la liaison à enfichage, laquelle entoure la partie de manipulation (15) et est mobile en direction axiale par-dessus la pièce d'accouplement (21) et l'élément de réception (23).

11. Dispositif de coupe (1 à 7, 14) selon l'une des revendications précédentes, **caractérisé en ce que** l'élément d'isolation (5) est constitué en céramique.

12. Dispositif de coupe (1 à 7, 14) selon l'une des revendications précédentes, **caractérisé en ce qu'**il est prévu, pour la coagulation de tissu en cas de saignements, une troisième électrode (11) et une quatrième électrode (12) destinées chacune au branchement à un générateur de haute fréquence, qui sont agencées à distance et de façon électriquement isolée l'une de l'autre sur la surface de l'élément de support (3) ou sur un corps d'isolation à plat (10), la troisième électrode (11) et la quatrième électrode (12) étant allongées chacune au moins localement et agencées l'une à côté de l'autre le long de leur extension longitudinale pour augmenter la surface efficace.

13. Dispositif de coupe (1 à 7, 14) selon la revendication 12, **caractérisé en ce que** la troisième électrode (11) et la quatrième électrode (12) sont conformées chacune en forme spiralée et agencées de manière à s'engager l'une dans l'autre.

14. Dispositif de coupe (1 à 7, 14) selon la revendication 13, **caractérisé en ce que** la troisième électrode (11) et la quatrième électrode (12) sont agencées en forme de méandres.
